# EUROPEAN PATENT APPLICATION

(11) **EP 2 837 389 A1**
(43) Date of publication of application: **18.02.2015**
(21) Application number: 13776214.2
(22) Date of filing: 12.04.2013
(51) Int. Cl.: A61K 45/00, A61K 9/127, A61K 31/7105, A61K 31/713, A61P 9/10, C12N 15/09, C12N 15/113

(54) **MEDICINAL COMPOSITION FOR TREATING INFARCTION**

(30) Priority: 12.04.2012 JP 2012090873
(71) Applicant: Gifu University, Gifu-shi, Gifu 501-1193 (JP); Otsuka Pharmaceutical Co., Ltd., Tokyo 101-8535 (JP)
(72) Inventor: MINATOGUCHI, Shinya, Gifu-shi Gifu 501-1193 (JP); AKAO, Yukihiro, Gifu-shi Gifu 501-1193 (JP)
(74) Representative: Hoffmann Eitle
(86) International application number: PCT/JP2013/061118
(87) International publication number: WO 2013/154192

(57) **Abstract**

The present invention provides a pharmaceutical composition for treating infarction, such as myocardial infarction or cerebral infarction, with a method that is different from conventional therapeutic methods that use a thrombolytic agent or balloon therapy.

An autophagy enhancer such as hsa-miR-145 is used as the active ingredient of the pharmaceutical composition for treating infarction. In particular, an autophagy enhancer such as hsa-miR-145 is used in a liposomal state as an active ingredient of the pharmaceutical composition for treating infarction.

SEQUENCE LISTING

<110>  GIFU UNIVERSITY
<110>  OTSUKA PHARMACEUTICAL CO., LTD.

<120>  Pharmaceutical composition for treating infarction

<130>  P13-053

<150>\201@JP 2012-090873
<151>\201@2012-04-12

<160>  3

<170>  PatentIn version 3.5

<210>  1
<211>  23
<212>  RNA
<213>  Homo sapiens

<400>  1
guccaguuuu cccaggaauc
ccu\201@\201@\201@\201@\201@\201@\201@\201@\201@\201@\201@\201@\201@\201@\201
@\201@\201@\201@\201@\201@\201@23

<210>  2
<211>  88
<212>  RNA
<213>  Homo sapiens

<400>  2
caccuugucc ucacggucca guuuucccag gaaucccuua gaugcuaaga uggggauucc\201@\201@60

uggaaauacu guucuugagg
ucaugguu\201@\201@\201@\201@\201@\201@\201@\201@\201@\201@\201@\201@\201@\201
@\201@\201@\201@\201@\201@ 88

<210>  3
<211>  19
<212>  RNA
<213>  Unknown
<223>  a base sequence of miRNA used in Example 3

<400>  3
guaggaguag
ugaaaggcc\201@\201@\201@\201@\201@\201@\201@\201@\201@\201@\201@\201@\201@
\201@\201@\201@\201@\201@\201@\201@\201@19

## Description

### Technical Field

The present invention relates to a pharmaceutical composition for treating infarction. More specifically, the present invention relates to the use of an "autophagy enhancer" that accelerates autophagy as an agent for treating infarction.

### Background Art

Myocardial infarction is a type of ischemic heart disease, and refers to a state in which the amount of blood flow in the coronary arteries of the heart decreases, the heart muscle becomes ischemic, and then the heart muscle dies. Decrease of blood flow in the coronary artery is mainly due to stenoses caused by a factor such as arteriosclerosis.

Examples of therapeutic methods for treating myocardial infarction include dissolving the blood clots with a thrombolytic agent (e.g., tPA), and balloon therapy, in which a catheter is inserted in the blood vessel to expand the blood vessel with a balloon. It is known that a therapeutic method in which a thrombolytic agent is administered via intravenous injection is simple, but reocclusion easily occurs. Furthermore, it is necessary to pay attention to patients with bleeding disorders such as gastric ulcer. Balloon therapy can achieve a high success rate in recanalization, and it can prevent restenosis by keeping a stent in the blood vessel after it is performed. However, restenosis may still occur. Furthermore, there is a limitation such that a balloon therapy can be conducted only in medical facilities that have a room for cardiac catheterization.

An miRNA is a single-stranded RNA consisting of about 21 to 25 nucleotides, and functions in regulation of gene expression. The miRNA is a small RNA derived from non-coding region encoded in the genome. Generally, an miRNA is produced from the gene transferred from a single or clustered miRNA precursor. More specifically, a pri-miRNA, which is a primary transcription from a gene, is transferred. Subsequently, in the stepwise processing from a pri-miRNA to a mature type of miRNA, a pre-miRNA consisting of about 70 to 80 nucleotides having a specific hairpin structure is produced from a pri-mRNA. Further, a mature type of miRNA is produced from pre-miRNA through the intervention of Dicer. It is believed that the miRNA is deeply involved in biological phenomenon, such as cell differentiation, proliferation, apoptosis, etc., that are essential for living things.

Among miRNAs, hsa-miR-145 is an miRNA that consists of 23 nucleotides and is believed to be involved in anti-cancer action, as well as the regulation of cell proliferation and differentiation of smooth muscle (Patent Literature 1, Non-patent Literature 1 and 2).

### Citation List

### Patent Literature

PTL 1: WO2009/105759

### Non-patent Literature

NPL 1: S. Noguchi et al., J. Vet. Med. Sci., 74(1), 1-8, 2012
NPL 2: Eric M. Small et al., Nature, 469, 336-342, 2011

### Summary of Invention

### Technical Problem

An object of the present invention is to provide a pharmaceutical composition for use in treating infarction, such as myocardial infarction or cerebral infarction, by a method that is different from a conventional therapeutic method using a thrombolytic agent and balloon therapy. In particular, the present invention aims to provide a pharmaceutical composition for treating infarcted tissue, wherein the pharmaceutical composition can selectively and noninvasively reach and treat the local region where a myocardial infarction or cerebral infarction occurs.

### Solution to Problem

The present inventors conducted intensive research to achieve the above objects and found that, by administering hsa-miR-145, which is an miRNA, to the site of infarcted tissue, the size of the site of infarction (infarct size) can be reduced, and that the miRNA has an action of accelerating autophagy. More specifically, they found that the miRNA can function as an "autophagy enhancer." Based on these findings, the present inventors believed that an autophagy enhancer, in particular, hsa-miR-145, is effective in the repair and amelioration of infarcted tissue, and useful as an active ingredient of gene therapy for treating infarcted tissue. The present invention has been thus accomplished.

More specifically, the present invention includes the following embodiments.

### (I) Pharmaceutical composition for treating infarction

(I-1) A pharmaceutical composition for treating infarction comprising an autophagy enhancer as an active ingredient.
(I-2) The pharmaceutical composition for treating infarction according to (I-1), wherein the autophagy enhancer is at least one member selected from the group consisting of hsa-miR-145, rapamycin, carbamazepine, sodium valproate, verapamil, loperamide, amiodarone, nimodipine, nitrendipine, niguldipine, primozide, calpastatin, calpeptin, clonidine, pilmenidine, 2',5'-dideoxyadenosine, minoxidil, penitrem A, fluspiriline, trifluoperazine, and trehalose.
(I-3) The pharmaceutical composition for treating infarction according to (1-1) or (I-2), comprising the autophagy enhancer in a liposomal state.
(I-4) The pharmaceutical composition for treating infarction according to any one of (I-1) to (I-3), which is in an intravenous administration.
(I-5) The pharmaceutical composition for treating infarction according to any one of (I-1) to (I-4), wherein the infarction is myocardial infarction, cerebral infarction, or spinal cord infarction.
(I-6) The pharmaceutical composition for treating infarction according to (1-5), wherein the myocardial infarction is severe.

### (II) Method for treating infarction

(II-1) A method for treating infarction, the method comprising administering an autophagy enhancer to a patient with infarction.
(II-2) The method for treating infarction according to (II-1), wherein the autophagy enhancer is at least one member selected from the group consisting of hsa-miR-145, rapamycin, carbamazepine, sodium valproate, verapamil, loperamide, amiodarone, nimodipine, nitrendipine, niguldipine, primozide, calpastatin, calpeptin, clonidine, pilmenidine, 2',5'-dideoxyadenosine, minoxidil, penitrem A, fluspiriline, trifluoperazine, and trehalose.
(II-3) The method for treating infarction according to (II-1) or (II-2), wherein the autophagy enhancer is administered in a liposomized state.
(II-4) The method for treating infarction according to any one of (II-1) to (II-3), comprising intravenously administering the autophagy enhancer.
(II-5) The method for treating infarction according to any one of (II-1) to (II-4), wherein the infarction patient has myocardial infarction, cerebral infarction, or spinal cord infarction.
(II-6) The method for treating infarction according to (II-5), wherein the myocardial infarction patient has a severe, large myocardial infarction.

In the inventions related to the method for treating infarction according to (II-1) to (II-6), the term "autophagy enhancer" can be replaced with "the pharmaceutical composition for treating infarction" defined by any one of (I-1) to (I-6) described above.

### (III) Novel use of autophagy enhancer

### [III-1] Autophagy enhancer for treating infarction:

(III-1-1) An autophagy enhancer for use in treating infarction.
(III-1-2) The autophagy enhancer according to (III-1-1), wherein the autophagy enhancer is at least one member selected from the group consisting of hsa-miR-145, rapamycin, carbamazepine, sodium valproate, verapamil, loperamide, amiodarone, nimodipine, nitrendipine, niguldipine, primozide, calpastatin, calpeptin, clonidine, pilmenidine, 2',5'-dideoxyadenosine, minoxidil, penitrem A, fluspiriline, trifluoperazine, and trehalose.
(III-1-3) The autophagy enhancer according to (III-1-1) or (III-1-2), wherein the autophagy enhancer is in a liposomal state.
(III-1-4) The method for treating infarction according to any one of (III-1-1) to (III-1-3), wherein the autophagy enhancer is intravenous administration.
(III-1-5) The autophagy enhancer according to any one of (III-1-1) to (III-1-4), wherein the infarction is myocardial infarction, cerebral infarction, or spinal cord infarction.
(III-1-6) The autophagy enhancer according to (III-1-5), wherein the myocardial infarction is severe.

### [III-2] Use of autophagy enhancer:

(III-2-1) Use of an autophagy enhancer for the manufacture of a pharmaceutical composition for treating infarction.
(III-2-2) Use of the autophagy enhancer according to (III-2-1), wherein the autophagy enhancer is at least one member selected from the group consisting of hsa-miR-145, rapamycin, carbamazepine, sodium valproate, verapamil, loperamide, amiodarone, nimodipine, nitrendipine, niguldipine, primozide, calpastatin, calpeptin, clonidine, pilmenidine, 2',5'-dideoxyadenosine, minoxidil, penitrem A, fluspiriline, trifluoperazine, and trehalose.
(III-2-3) The use according to (III-2-1) or (III-2-2), wherein the autophagy enhancer is in a liposomal state.
(III-2-4) The use according to any one of (III-2-1) to (III-2-3), wherein the pharmaceutical composition for treating infarction is in an intravenous administration.
(III-2-5) The use according to any one of (III-2-1) to (III-2-4), wherein the infarction is myocardial infarction, cerebral infarction, or spinal cord infarction.
(III-2-6) The use according to (III-2-5), wherein the myocardial infarction is severe.

### Advantageous Effects of Invention

The pharmaceutical composition for treating infarction of the present invention is characterized in that it comprises an autophagy enhancer, in particular, hsa-miR-145, as an active ingredient. The pharmaceutical composition for treating infarction of the present invention can reduce the infarct size by administering an autophagy enhancer, in particular, hsa-miR-145 (i.e., an active ingredient), to the infarcted site; therefore, it can be effectively used for treating infarction. As is clear from Example 2 described later, the pharmaceutical composition of the present invention has a remarkable effect in reduction of the infarct size in myocardial infarction, in particular, in severe, large myocardial infarction.

Severe, large myocardial infarction is a disease with an unfavorable life prognosis, since the progression of left ventricular remodeling results in heart failure. The pharmaceutical composition for treating infarction of the present invention can favorably improve the life prognosis after the treatment of the severe, large myocardial infarction.

### Brief Description of Drawings

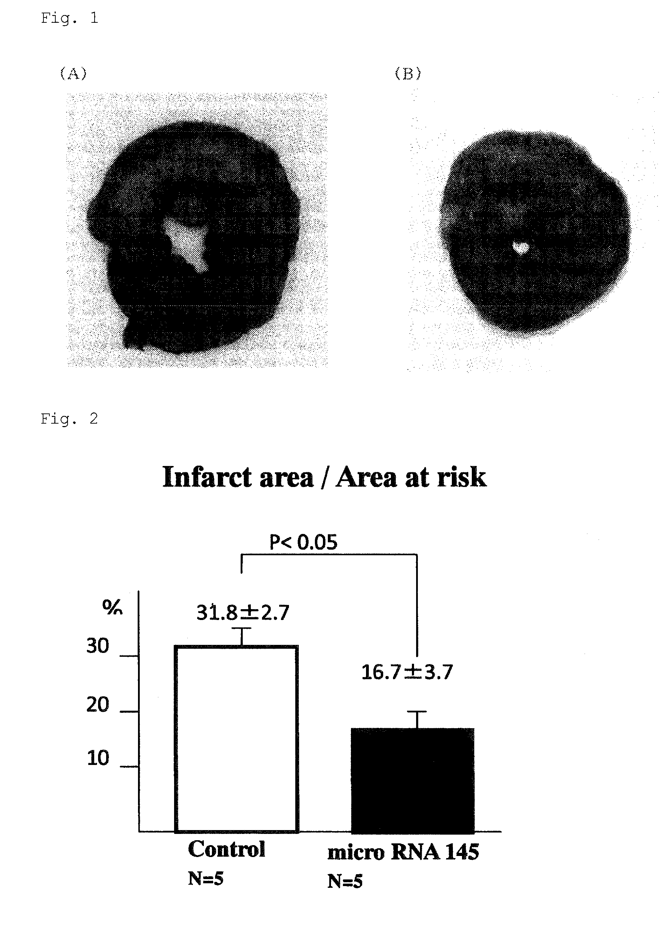

Fig. 1 shows stained images of the heart tissue of a severe, large myocardial infarction animal model (rabbit) stained with Evans Blue and TTC (Example 2). Fig. 1(A) shows the stained image of the heart tissue of a severe, large myocardial infarction animal model (control group) to which physiological saline was intravenously administered instead of an hsa-miR-145-containing liposome. Fig. 1(B) shows the stained image of the heart tissue of a severe, large myocardial infarction animal model (hsa-miR-145 administered group) to which an hsa-miR-145-containing liposome was intravenously administered.
Fig. 2 is a graph that indicates the infarct size in the hearts of the hsa-miR-145 administered group (n=5) and the control group (n=5) as a proportion (%) of the infarct area of the heart relative to the area at risk of the entire heart.
Fig. 3 shows an electrophoretic profile of the Western blotting performed in Example 3. Fig. 3 shows expressions of each protein (FRS2, KLF5, Myocardin, LC3B, p-Akt, Akt, p-ERK1/2, ERK1/2, and β-actin) in H9c2 cells treated with hsa-miR-145.
Fig. 4 shows images of H9c2 cells treated with hsa-miR-145 observed with an electron microscope. Fig. 4(A) clearly indicates that autophagosomes are formed in the cell, and Fig. 4(B) clearly indicates that autolysosomes are formed in the cell (Example 3).

### Description of Embodiments

The pharmaceutical composition for treating infarction of the present invention is characterized in that it comprises an autophagy enhancer as an active ingredient.

Autophagy is a self-purification action that cells inherently possess. It is a mechanism in which cellular components that become old and unnecessary are wrapped in a vesicle-like structure (an autophagosome) together with cytoplasms, and degraded via lysosomal enzymes. There are two types of autophagy: one is basic autophagy, which is necessary to maintain intracellular metabolism, homeostasis, etc.; and the other is stress-induced autophagy. By the action of autophagy, intracellular accumulation of abnormal proteins can be suppressed. Furthermore, when an excessive amount of protein is produced, the intracellular nutrient environment is deteriorated, or pathogenic microorganisms enter the cytoplasm, etc., autophagy functions to remove or purify them. As described above, autophagy is involved in the maintenance of homeostasis in a living body. Autophagy is also induced when the cells fall into starvation, or pathological conditions such as ischemia or inflammation.

In the present invention, "autophagy enhancer" indicates a substance that can accelerate the aforesaid "autophagy," which is the self-purification action of cell. Specific examples thereof include hsa-miR-145, a type of miRNA whose ability to accelerate autophagy is demonstrated in the Examples below; and substances conventionally known to have an action of accelerating autophagy, such as rapamycin, carbamazepine, sodium valproate, verapamil, loperamide, amiodarone, nimodipine, nitrendipine, niguldipine, primozide, calpastatin, calpeptin, clonidine, pilmenidine, 2',5'-dideoxyadenosine, minoxidil, penitrem A, fluspiriline, trifluoperazine, and trehalose (S. Sarkar et al., Cell Death and Differentiation 16, 46-56, 2009). Note that these autophagy enhancers may be used as an active ingredient of the pharmaceutical composition for treating infarction of the present invention singly or in any combination of two or more, as long as they do not adversely affect the action of autophagy acceleration. Preferably, hsa-miR-145, or a combination of hsa-miR-145 with one, or two or more other autophagy enhancers is used.

Hsa-miR-145 is an oligonucleotide known as a human-derived micro RNA, and is registered as Accession No. MI0000461 in the microRNA database (miRBase) (http://www.mirbase.org/) (Entrez Gene: 406937, HGNC: 31532).

SEQ ID NO: 1 shows the nucleotide sequence of hsa-miR-145. SEQ ID NO: 2 shows the nucleotide sequence of a primary miRNA (pri-miRNA) having a hairpin loop structure, which is a molecule before it is processed into hsa-miR-145.

Hsa-miR-145 can be synthesized by a known nucleic acid synthetic process based on this nucleotide sequence (SEQ ID NO: 1); hsa-miR-145 is also commercially available from Applied Biosystems, etc. When used for the pharmaceutical composition for treating infarction, regardless of the production method of the hsa-miR-145, the hsa-miR-145 may be artificially chemosynthesized, biochemically synthesized, synthesized in a living body, or produced by double-stranded RNA (pri-miRNA) having 80 or more nucleotides being decomposed in or outside of the body.

The pharmaceutical composition of the present invention can be produced by combining the aforesaid autophagy enhancer as an active ingredient with, depending on the administration route, pharmaceutically acceptable carriers or additives ordinarily used for medicaments. When a nucleic acid such as, in particular, hsa-miR-145, is used as the autophagy enhancer, the pharmaceutical composition of the present invention can be generally produced by combining it with pharmaceutically acceptable carriers or additives used for the production of gene therapy agents.

The administration route is not limited as long as the autophagy enhancer can be delivered to the affected site (infarcted tissue), and may be administered orally or non-orally (e.g., intravenous administration, direct administration to the affected site, intramuscular administration, subcutaneous administration, intradermal administration, mucosal administration, rectal administration, and transpulmonary administration). Among these, intravenous administration by injection or infusion, and direct administration to the affected site are preferable.

In the case of an injection preparation or infusion preparation, carriers or additives that can be used with the autophagy enhancer include various carriers or additives ordinarily used for injection preparations or infusion preparations. Examples of the carriers include distillated water; an aqueous solution containing sodium chloride or a mixture of sodium chloride with an inorganic salt; an aqueous solution containing a saccharide such as mannitol, lactose, dextran, or glucose; an aqueous solution containing an amino acid such as glycine or arginine; an aqueous solution containing an organic acid and a saccharide such as glucose; and an aqueous solution containing a salt and a saccharide such as glucose. These preparations may be formed into a solution, suspension, or emulsion by using, if necessary, auxiliary agents such as an osmotic agent, a pH adjuster, vegetable oil, and/or a surfactant. Furthermore, a pharmaceutically acceptable anti-oxidant or preservative may be used as an additive. Note that the injection preparation and infusion preparation may be formed, by powdering (spray-drying) or freeze-drying, into a solid preparation that is dissolved before use.

The pharmaceutical composition of the present invention may be in a form that contains an unmodified autophagy enhancer dissolved in a carrier for injection preparation such as distillated water; or the pharmaceutical composition of the present invention may be in a form that contains an autophagy enhancer in a liposomal state. Here, the term liposomal encompasses both the state in which the autophagy enhancer is enclosed in a liposome (encapsulated in a liposome), and the state in which the autophagy enhancer is attached on the surface of liposome.

The liposome is not limited as long as it can enclose (encapsulate) the autophagy enhancer of the present invention therein, or as long as the autophagy enhancer can be attached on its surface. There is no particular limitation to the liposome; however, when hsa-miR-145 is used as the autophagy enhancer, because the hsa-miR-145 is anionic, examples of the usable liposomes include those having a positive electric charge such as Lipofectamine, Lipofectin, Cellfectin, and other positively charged liposomes; and liposomes comprising components such as calcium phosphate, dextran, and/or polyethylene glycol. These liposomes are also commercially available; for example, Lipofectamine can be purchased from Invitrogen Coporation, etc., and FuGENE 6 Transfection Reagent can be purchased from Roche Diagnostics.

A method for encapsulating a drug in a liposome is known. The autophagy enhancer of the present invention can be encapsulated in a liposome according to such a known method. For example, when the commercially available liposomes mentioned above are used, the preparation can be conducted according to the manufacturer's manual. If commercially available liposomes are not used, a liposome encapsulating an autophagy enhancer therein can be obtained by, for example, forming a liposome using a solution containing an autophagy enhancer and lipids containing phosphatidylcholines, phosphatidylethanolamine, phosphatidic acids or long-chain alkyl phosphates, gangliosides, glycolipids or phosphatidylglycerols and cholesterols.

The mean particle diameter of the liposome is preferably 150 nm or less, more preferably 140 nm or less, still more preferably 120 nm or less, and furthermore preferably 100 nm or less in consideration of passing it through the capillary. The mean particle diameter is preferably 30 nm or more, more preferably 50 nm or more, and further more preferably 70 nm or more. Here, the mean particle diameter refers to the value of the particle diameter at 50% in the cumulative distribution determined by the laser diffraction scattering method.

In addition to injection preparations or infusion preparations, the pharmaceutical composition of the present invention may also take the form of oral administrations such as solid preparations, e.g., tablets, capsules, granules, and powder. In the production of such solid preparations, in accordance with an ordinary method, pharmaceutically acceptable carriers or additives may be added. Examples thereof include, but are not limited to, excipients, binders, disintegrators, solubilizing agents, lubricants, anti-oxidants, preservatives, pH adjusters, coloring agents, and flavoring agents.

The amount of the autophagy enhancer contained in the pharmaceutical composition of the present invention varies depending on the administration route of the pharmaceutical composition of the present invention, and the type of autophagy enhancer. However, when hsa-miR-145 is used as an autophagy enhancer, the pharmaceutical composition of the present invention may contain the hsa-miR-145 in an amount of 10 to 1000 µg, preferably 50 to 160 µg, and more preferably 70 to 80 µg per dose. When another autophagy enhancer is used, the amount thereof may be suitably selected based on the above.

The pharmaceutical composition of the present invention can be used as an agent for treating infarction of patients having myocardial infarction, cerebral infarction, or spinal cord infarction. The pharmaceutical composition of the present invention is preferably used for treating myocardial infarction, and more preferably treating severe, large myocardial infarction.

The dose of the pharmaceutical composition of the present invention may be suitably selected depending on the types or severity of the infarction such as myocardial infarction, cerebral infarction, or spinal cord infarction; and the age, body weight of the patient. The dose is preferably the amount suitable for regenerating or repairing at least some portion of infarcted tissue, and this amount is referred to "a pharmaceutically effective amount" in the present invention. The following is an approximation of the effective amount: as the dose of the autophagy enhancer per administration, for example, when hsa-miR-145 is used, the content of hsa-miR-145 is generally 10 to 1000 µg, preferably 50 to 160 µg, and more preferably 70 to 80 µg. When other autophagy enhancers are used, the amount thereof can be suitably selected based on the above amount.

The frequency of administration of the pharmaceutical composition of the present invention may be once or several times per day, and is suitably selected depending on the conditions of the infarction patient.

### Examples

The present invention is described in more detail below with reference to Examples and the like. However, the present invention is not limited to these Examples.

### Example 1: Preparation of hsa-miR-145-containing liposome

Hsa-miR-145 (5 nM; obtained from Applied Biosystems) was dissolved in 100 µl of RNase-free distillated water. 400 µl of reduced serum medium (Opti-MEM (GIBCO)) was added thereto, followed by mixing by shaking with a vortex mixer for 30 seconds. Thereafter, 10 µl of Lipofectamine RNAiMax (Invitrogen) was added to the mixture, followed by mixing by shaking with a vortex mixer for another 30 seconds. Subsequently, the mixture thus obtained was incubated at room temperature for 10 minutes. 300 µl of Opti-MEM was added thereto to a total volume of 800 µl. The hsa-miR-145-containing liposome thus prepared was used in Example 2 described below.

### Example 2. Application to myocardial infarction and therapeutic effect thereof

The hsa-miR-145-containing liposome prepared in Example 1 above was administered to the infarcted site of an animal model having a severe, large myocardial infarction so as to evaluate the therapeutic effect of hsa-miR-145 on infarction.

### 1. Test Method

A pathological animal model having a severe, large myocardial infarction was prepared using a Japanese white rabbit (male, body weight: about 2 kg), and subjecting its coronary artery to ischemia for 30 minutes.

The hsa-miR-145-containing liposome prepared in Example 1 was intravenously administered to each of the pathological animal models (n=5) in an amount of 0.035 mg per kg of body weight. 14 days after the administration, the rabbits were sacrificed under anesthesia, and their hearts were extracted (hsa-miR-145 administered group). For the control group, instead of the hsa-miR-145-containing liposome, 0.5 ml of physiological saline per kg of body weight was intravenously administered to pathological animal models (n=5) having severe, large myocardial infarction. 14 days after the administration, the rabbits were sacrificed under anesthesia, and their hearts were extracted.

Each group of hearts (the hsa-miR-145 administered group, and the control group) thus extracted was stained with Evans Blue Dye so as to determine the ischemic region and the non-ischemic region. The hearts were stained with TTC so as to determine the infarct area.

Using these stains as indexes, the size of the infarct area was calculated as the proportion (%) to the area at risk 100% (potential ischemic area) of the entire heart.

### 2. Test Results

Fig. 1 shows the stained tissue in a stenotic site in the heart (obstructed site in the heart) in each group (hsa-miR-145 administered group and the control group). In Fig. 1, (A) shows the stained image of the heart tissue of the control group, and (B) shows the stained image of the heart tissue of the hsa-miR-145 administered group. The region stained blue with Evans Blue is the non-ischemic region; the region that was not stained blue is the ischemic region; and the region stained gray with TTC is the infarct area.

Fig. 2 shows a comparison in the infarct size in each heart between the hsa-miR-145 administered group (n=5) and the control group (n=5). As is clear from Fig. 2, the infarct size in the heart in the hsa-miR-145 administered group was significantly smaller than the infarct size in the heart of the control group to which hsa-miR-145 was not administered. These results confirmed that hsa-miR-145 has an action that reduces infarct size.

The measurement of the expression amount of hsa-miR-145 can be performed with a known method. A specific method is as follows:
The tissues are separately collected from the infarct site, boundary site, and normal site in an extracted heart. The collected tissues are homogenized and dissolved using TRIzol® Reagent (Applied Biosystems; AB) to extract RNA. Thereafter, RT is conducted with a TaqMan miRNA Reverse Transcription Kit (AB), and the expression of hsa-miR-145 is detected by real-time PCR using a TaqMan miRNA primer (AB).

The forward primer, reverse primer, and probe used in the RT-PCR can be suitably selected based on a known method.

### Example 3. Verification of mechanism of action of hsa-miR-145

In Example 2 described above, it was confirmed that hsa-miR-145 was effective for the reduction of the myocardial infarction foci, and preservation of the cardiac function. Therefore, the mechanisms of the action of hsa-miR-145 were verified.

### (1) Test Method

### (1-1) Preparation of Sample

H9c2 cells, which are derived from rat heart, were transfected with the hsa-miR-145-containing liposome prepared in Example 1 above. Specifically, the hsa-miR-145-containing liposome was prepared to have a final hsa-miR-145 concentration of 20 nM or 40 nM, after which H9c2 cells were cultured in the presence of the hsa-miR-145-containing liposome at 37°C for 72 hours.

After culturing, total protein was extracted from the H9c2 cells thus obtained. Specifically, the cultured H9c2 cells were scraped off from the culture dish using a cell scraper (Sumitomo Bakelite Co., Ltd., Japan), and then suspended in Phosphate Buffered Saline (PBS(-); Takara, Japan). The result was placed in a 15-ml centrifuge tube, and then centrifuged at 2000 rpm for 3 minutes. After the centrifugation, the supernatant was removed, and Lysis Buffer (1% NP-40, 0.1% deoxycholic acid, 0.1% SDS, 150 mM NaCl, 1 mM EDTA) and 1% proteinase inhibitor cocktail (Sigma-Aldrich Co.) were added to the precipitate. The mixture was stirred with a vortex mixer to completely dissolve the cells. After being allowed to stand still on ice for 20 minutes, the result was centrifuged at 4°C at 12000 rpm for 20 minutes, and the supernatant thereof was collected to be used as a protein extract. The protein concentration of the protein extract was measured with a DC Protein Assay Kit (Bio-Rad; Hercules, CA, USA) according to the protocol of the kit using a spectrophotometer (BioPhotometer Plus, Eppendorf AG; Hamburg, Germany).

The protein extract (total protein amount: 100 µg) prepared above and 20 µl of 5×SDS solution (0.25 M Tris-HCl (pH 6.8), 10% SDS, 0.5% Bromophenol Blue, 0.5% M DTT, 50% glycerol) were added and mixed in a microtube. Sterile distilled water was added thereto to a total amount of 100 µl. Subsequently, the mixture was heated at 98°C for 5 minutes with a block incubator (Astec; Fukuoka, Japan), and then quenched on ice for 5 minutes.

### (1-2) Western blotting

The above-described protein treated with SDS was electrophoresed using an XV Pantera System (DRC Co., Ltd.; Tokyo, Japan) or Easy Separator® (Wako Pure Chemical Industries, Ltd.) After electrophoresis, the sample was blotted on a PVDF membrane (PerkinElmer Life Science; Boston, MA, USA) using a Trans-Blot SD Semi-Dry Transfer Cell (Bio-Rad). The PVDF membrane was hydrophilized with methanol beforehand, immersed in a blotting buffer (48 mM Tris, 40 mM glycine), and then shaken before use. Blotting was performed at 15 V, 370 mA, for 40 minutes.

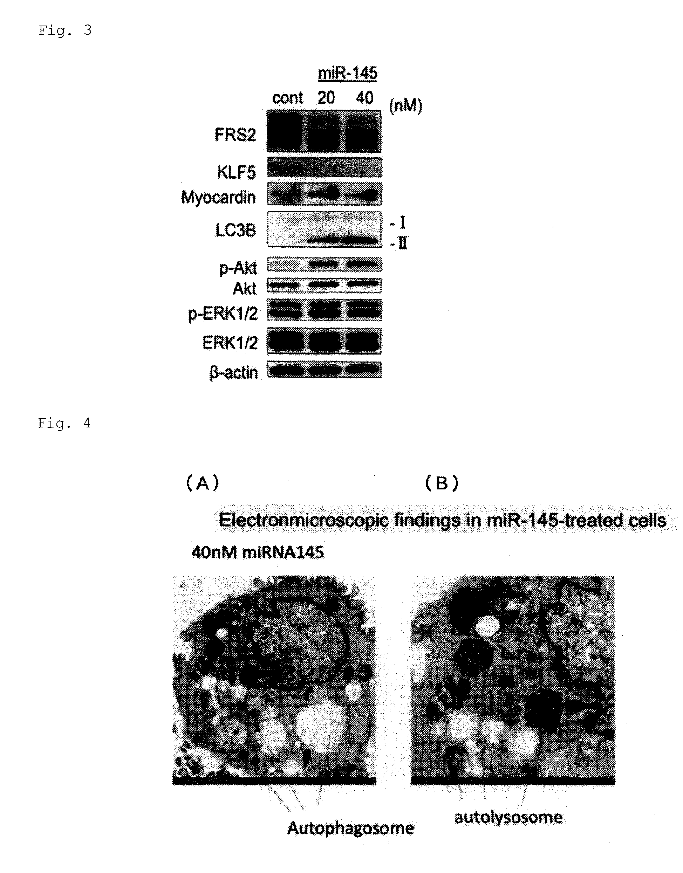

After the completion of blotting, the result was immersed in Tris-buffered saline-Tween 20 (TBS-T) (20 mM tris-HCl (pH 7.5), 150 mM NaCl, 0.1% Tween 20) to which 5% skim milk (Cell Signaling; Danvers, MA, USA) had been added. Then, blocking was performed at room temperature while shaking for 1 hour. After washing with TBS-T, the resultant was immersed in a primary antibody solution diluted with a primary antibody diluent (2% bovine serum albumin, 0.01% sodium azide-added TBS-T) at 4°C overnight. Antibodies of each of the proteins shown in Fig. 3 (FRS2, KLF5, Myocardin, LC3B, p-Akt, Akt, p-ERK1/2, ERK1/2, and β-actin) were used as the primary antibody. The next day, the resultant was washed with TBS-T, immersed in a secondary antibody solution diluted with 5% skim milk, shaken for 1 hour, and then washed with TBS-T. A monoclonal anti-mouse or anti-rabbit horseradish-labeled antibody (#7076 or #7074, Cell Signaling Technology, Inc.) (1:4000) was used as the secondary antibody.

Subsequently, color development was performed using Western Lightning® ECL Pro (PerkinElmer), and detection was performed by chemiluminescence using an ImageQuant LAS 4000 Mini (GE Healthcare Japan; Tokyo, Japan).

As a control for loading, detection was performed using the same membrane and using an anti-human β-actin mouse monoclonal antibody (AC-15, Sigma-Aldrich) (1:4000) as a primary antibody. After sanitizing at room temperature for 1 hour, the detection was performed according to the protocol described above.

### (1-3) Comparative Experiment

An experiment was conducted in the same manner as Example 3(1-1) and Example 3(1-2) above, except that miRNA having a nucleotide sequence of 5'-GUAGGAGUAGUGAAAGGCC-3' (Dharmacon, Inc.) (SEQ ID NO: 3) was used instead of hsa-miR-145.

### (2) Test Results

Fig. 3 shows the electrophoretic profile of the Western blotting performed in the experiment above. In Fig. 3, control in the electrophoretic profile indicates the results of the Comparative Experiment above.

As is clear from these results, a remarkable conversion from LC3-BI to LC3-BII and acceleration of PI3K/Akt (p-Akt) signaling were observed in a cell into which hsa-miR-145 was introduced. In contrast, reduction in expression was observed in both KLF5 and FRS2 (fibroblast growth factor receptor substrate 2).

Fig. 4 shows H9c2 cells treated with hsa-miR-145 having a final concentration of 40 nM, observed with an electron microscope. As shown in Fig. 4, the shapes of autophagosomes (Fig. 4 (A)) and autolysosomes (Fig. 4 (B)) were observed in the cells.

### Sequence Listing Free Text

SEQ ID NO: 1 shows the nucleotide sequence of hsa-miR-145. SEQ ID NO: 2 shows the nucleotide sequence of the primary miRNA having a hairpin loop structure, which is a precursor of hsa-miR-145. SEQ ID NO: 3 shows the nucleotide sequence of the miRNA used in the Comparative Experiment of Example 3.

## Claims

1. A pharmaceutical composition for treating infarction comprising an autophagy enhancer as an active ingredient.

2. The pharmaceutical composition for treating infarction according to claim 1, wherein the autophagy enhancer is hsa-miR-145.

3. The pharmaceutical composition for treating infarction according to claim 1 or 2, comprising the autophagy enhancer in a liposomal state.

4. The pharmaceutical composition for treating infarction according to any one of claims 1 to 3, which is in an intravenous administration.

5. The pharmaceutical composition for treating infarction according to any one of claims 1 to 4, wherein the infarction is myocardial infarction, cerebral infarction, or spinal cord infarction.

6. A method for treating infarction, the method comprising administering an autophagy enhancer to a patient with infarction.

7. The method for treating infarction according to claim 6, wherein the autophagy enhancer is hsa-miR-145.

8. The method for treating infarction according to claim 6 or 7, wherein the autophagy enhancer is administered in a liposomal state.

9. The method for treating infarction according to any one of claims 6 to 8, wherein the autophagy enhancer is intravenous administration.

10. An autophagy enhancer for use in treating infarction.

11. The autophagy enhancer according to claim 10, wherein the autophagy enhancer is hsa-miR-145.

12. Use of an autophagy enhancer for the manufacture of a pharmaceutical composition for treating infarction.

13. The use according to claim 12, wherein the autophagy enhancer is hsa-miR-145.
